(19) Europäisches Patentamt
European Patent Office
Office européen des brevets

(11) EP 2 911 704 B1

(12) **EUROPEAN PATENT SPECIFICATION**

(45) Date of publication and mention of the grant of the patent:
**16.10.2019 Bulletin 2019/42**

(21) Application number: **13780159.3**

(22) Date of filing: **24.10.2013**

(51) Int Cl.:
***A61K 49/10*** *(2006.01)*

(86) International application number:
**PCT/EP2013/072269**

(87) International publication number:
**WO 2014/064194 (01.05.2014 Gazette 2014/18)**

(54) **HYPERPOLARIZED 2-OXOGLUTARATE AS METABOLIC AGENT IN MAGNETIC RESONANCE**

HYPERPOLARISIERTES 2-OXOGLUTARAT ALS METABOLISCHES MITTEL IN MAGNETISCHER RESONANZ

2-OXOGLUTARATE HYPERPOLARISÉ EN TANT QU'AGENT MÉTABOLIQUE EN RÉSONANCE MAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.10.2012 EP 12189901**

(43) Date of publication of application:
**02.09.2015 Bulletin 2015/36**

(73) Proprietor: **Bracco Imaging S.p.A.**
**20134 Milano (IT)**

(72) Inventors:
- **JENSEN, Pernille Rose**
  **DK-22100 KBH Copenhagen (DK)**
- **KARLSSON, Magnus**
  **S-21243 Malmö (SE)**
- **LERCHE, Mathilde**
  **DK-1903 Frederiksberg C (DK)**
- **CABELLA, Claudia**
  **I-10080 Torino (IT)**
- **CAMINITI, Lara**
  **I-10010 Ivrea (IT)**
- **COLOMBO SERRA, Sonia**
  **I-10010 Ivrea (IT)**
- **MIRAGOLI, Luigi**
  **I-10010 Ivrea (IT)**
- **POGGI, Luisa**
  **I-10010 Ivrea (IT)**
- **VENTURI, Luca**
  **I-10010 Ivrea (IT)**
- **TEDOLDI, Fabio**
  **I-10010 Ivrea (IT)**

(74) Representative: **Ravizza, Claudio**
**Bracco Suisse S.A.**
**Intellectual Property Department**
**31, route de la Galaise**
**1228 Plan-les-Ouates (CH)**

(56) References cited:
**WO-A1-2011/124672 WO-A1-2013/053839**
**US-A1- 2007 196 280**

- **GALLAGHER FERDIA A ET AL: "Detection of tumor glutamate metabolism in vivo using (13)C magnetic resonance spectroscopy and hyperpolarized [1-(13)C]glutamate.", MAGNETIC RESONANCE IN MEDICINE : OFFICIAL JOURNAL OF THE SOCIETY OF MAGNETIC RESONANCE IN MEDICINE / SOCIETY OF MAGNETIC RESONANCE IN MEDICINE JUL 2011, vol. 66, no. 1, July 2011 (2011-07), pages 18-23, XP002693201, ISSN: 1522-2594 cited in the application**
- **Chaumeil M.M. et al: "Non-invasive assessment of IDH status in glioblastoma using dynamic 13C MRS of hyperpolarized alpha-ketoglutarate and hyperpolarized pyruvate", The 9th Annual Imaging Research Symposium, University of California, San Francisco, October 30th 2012,poster abstract , XP002693200, Retrieved from the Internet: URL:http://www.radiology.ucsf.edu/sites/all/files/filemanager/research/imaging_research_symposium/Poster-Presentations2012final.pdf [retrieved on 2012-03-04] cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 911 704 B1

## Description

FIELD OF THE INVENTION

The invention relates to the field of Magnetic Resonance (MR), in particular to a method of $^{13}$C-MR detection using a diagnostic medium comprising hyperpolarized $^{13}$C-oxoglutarate.

BACKGROUND OF THE INVENTION

Magnetic resonance imaging (MRI) is a technique that has become particularly attractive to physicians as images of a patient's body or parts thereof can be obtained in a non-invasive way and without exposing the patient and the medical personnel to potentially harmful radiation such as X-rays. Because of its high quality images and good spatial and temporal resolution, MRI is a favourable imaging technique for imaging soft tissue and organs. MRI may be carried out with or without MR contrast agents. However, contrast-enhanced MRI usually enables the detection of much smaller tissue changes which makes it a powerful tool for the detection of early stage tissue changes like for instance small tumors or metastases.

MRI using hyperpolarized molecules is an emerging technique. WO 9935508 discloses a method of MR investigation of a patient using a hyperpolarized solution of a high $T_1$ agent as MRI contrast agent. The term "hyperpolarization" means enhancing the nuclear polarization of the NMR active nuclei present in the agent, i.e. nuclei with non-zero nuclear spin, preferably $^{13}$C- or $^{15}$N-nuclei, and thereby amplifying the MR signal intensity by a factor of hundred and more. When using a hyperpolarized $^{13}$C- and/or $^{15}$N-enriched high $T_1$ agent, there will be essentially no interference from background signals as the natural abundance of $^{13}$C and/or $^{15}$N is negligible and thus the image contrast will be advantageously high. The main difference between conventional MRI contrast agents and these hyperpolarized high $T_1$ agents is that in the former changes in contrast are caused by affecting the relaxation times of water protons in the body whereas the latter class of agents can be regarded as non-radioactive tracers, as the signal obtained arises solely from the agent. When hyperpolarization is obtained via a microwave assisted transfer between unpaired electrons and the nuclei used as MR probes, the techniques is referred as Dynamic Nuclear Polarization (DNP).

A variety of possible high $T_1$ agents for use as MR imaging agents are disclosed in WO 9935508, including non-endogenous and endogenous compounds. As examples of the latter, intermediates in normal metabolic cycles are mentioned which are said to be preferred for imaging metabolic activity. By in vivo imaging of metabolic activity, information of the metabolic status of a tissue may be obtained and said information may for instance be used to discriminate between healthy and diseased tissue.

For example, WO 2009077575 discloses a method of $^{13}$C-MR detection using an imaging medium comprising hyperpolarized $^{13}$C-fumarate, in order to investigate both the citric acid and the urea cycles by detecting $^{13}$C-malate and optionally $^{13}$C-fumarate and/or $^{13}$C-succinate signals. The metabolic profile generated in a preferred embodiment of the method provides information about the metabolic activity of the body, part of the body, cells, tissue, body sample under examination and said information may be used in a subsequent step for, e.g. identifying diseases. Such a disease is preferably cancer since tumor tissue is usually characterized by an altered metabolic activity. As a technical aspect, if the compounds to be polarized crystallize upon freezing or cooling their solution, a glass-forming additive must be added to the solution.

WO 2011124672, in the name of the Applicant, provides an alternative method for the hyperpolarization of molecules of biological interest, particularly for those which are part of metabolic pathways, such as tricarboxylic acid cycle, glycolysis, beta-oxidation, urea cycle etc. It was found that the use of a stable hyperpolarized precursor which can be readily transformed into the desired hyperpolarized substrate upon dissolution in an aqueous carrier is particularly advantageous, since this helps to avoid the use of any glass-forming additive.

Dynamic nuclear polarization (DNP) has been applied recently to magnetic resonance spectroscopy (MRS) in solution, where it can be used to produce a large increase in sensitivity. Using this technique, the metabolism of several $^{13}$C-labeled compounds has been observed and used to estimate rate constants for specific enzyme-catalyzed reactions in vitro and in vivo (Day SE, Kettunen MI, Gallagher FA, Hu DE, Lerche M, Wolber J, Golman K, Ardenkjaer-Larsen JH, Brindle KM. Detecting tumor response to treatment using hyperpolarized 13C magnetic resonance imaging and spectroscopy. Nat Med 2007; 13:1382-1387; Gallagher FA, Kettunen MI, Hu DE, Jensen PR, Zandt RI, Karlsson M, Gisselsson A, Nelson SK, Witney TH, Bohndiek SE, Hansson G, Peitersen T, Lerche MH, Brindle KM. Production of hyperpolarized [1,4-13C2]malate from [1,4-13C2]fumarate is a marker of cell necrosis and treatment response in tumors. Proc Natl Acad Sci USA 2009; 106:19801-19806). Furthermore, for some hyperpolarized $^{13}$C-labeled substrates there is sufficient signal for the spatial distribution of both the substrate and its metabolites to be imaged in vivo. As some of these substrates have already been administered at relatively high concentrations in the clinic, this technique has the potential to be translated into clinical applications. To date, the most studied reactions have been those involving hyperpolarized [1-$^{13}$C]pyruvate: the hyperpolarized label can be exchanged with either endogenous lactate or alanine, or alternatively it

can be irreversibly converted to carbon dioxide, which is subsequently converted to bicarbonate in the reaction catalyzed by carbonic anhydrase. These metabolic reactions have been observed in tumors, in cardiac tissue and in the liver (Merritt ME, Harrison C, Storey C, Jeffrey FM, Sherry AD, Malloy CR. Hyperpolarized 13C allows a direct measure of flux through a single enzyme-catalyzed step by NMR. Proc Natl Acad Sci USA 2007;104:19773-19777; Schroeder MA, Swietach P, Atherton HJ, Gallagher FA, Lee P, Radda GK, Clarke K, Tyler DJ. Measuring intracellular pH in the heart using hyperpolarized carbon dioxide and bicarbonate: a 13C and 31P MRS study. Cardiovasc Res 2010;86:82-91; Hu S, Chen AP, Zierhut ML, Bok R, Yen YF, Schroeder MA, Hurd RE, Nelson SJ, Kurhanewicz J, Vigneron DB. In vivo carbon-13 dynamic MRS and MRSI of normal and fasted rat liver with hyperpolarized 13C-pyruvate. Mol Imaging Biol 2009;11:399-407.).

Recently, other endogenous molecules have been successfully hyperpolarized: tumor pH has been measured in vivo from the relative concentrations of $^{13}$C-labeled bicarbonate and carbon dioxide following the injection of hyperpolarized $^{13}$C-labeled bicarbonate (Gallagher FA, Kettunen MI, Day SE, Hu DE, Ardenkjaer-Larsen JH, Zandt R, Jensen PR, Karlsson M, Golman K, Lerche MH, Brindle KM. Magnetic resonance imaging of pH in vivo using hyperpolarized 13C-labelled bicarbonate. Nature 2008;453:940-943); elevated levels of hyperpolarized malate have been demonstrated in necrotic tumor tissue in vivo following the injection of hyperpolarized $^{13}$C-labeled fumarate (Gallagher FA, Kettunen MI, Hu DE, Jensen PR, Zandt RI, Karlsson M, Gisselsson A, Nelson SK, Witney TH, Bohndiek SE, Hansson G, Peitersen T, Lerche MH, Brindle KM. Production of hyperpolarized [1,4-13C2]malate from [1,4-13C2]fumarate is a marker of cell necrosis and treatment response in tumors. Proc Natl Acad Sci USA 2009; 106:19801-19806); the metabolism of glutamine to glutamate, catalyzed by the mitochondrial enzyme glutaminase, has been observed following administration of hyperpolarized $^{13}$C-labeled glutamine to cells in vitro (Gallagher FA, Kettunen MI, Day SE, Lerche M, Brindle KM. 13C MR spectroscopy measurements of glutaminase activity in human hepatocellular carcinoma cells using hyperpolarized 13C-labeled glutamine. Magn Reson Med 2008;60:253-257); the organ-specific metabolism of hyperpolarized $^{13}$C-labeled acetate to acetyl-CoA and acetyl carnitine has been observed in vivo (Jensen PR, Peitersen T, Karlsson M, In 't Zandt R, Gisselsson A, Hansson G, Meier S, Lerche MH. Tissue-specific short chain fatty acid metabolism and slow metabolic recovery after ischemia from hyperpolarized NMR in vivo. J Biol Chem 2009;284:36077-36082), and the metabolism of branched chain amino acids has been observed in tumors following the addition of hyperpolarized $^{13}$C-labeled α-ketoisocaproate (Karlsson M, Jensen PR, Zandt R, Gisselsson A, Hansson G, Duus JO, Meier S, Lerche MH. Imaging of branched chain amino acid metabolism in tumors with hyperpolarized 13C ketoisocaproate. Int J Cancer 2010;127:729-736.10).

Glutamate is central to cellular metabolism, and its transamination product, α-ketoglutarate (a-KG) (or 2-oxoglutarate), is an intermediate in the citric acid cycle.

The detection of tumor α-KG levels has assumed particular importance recently with the demonstration that mutations in isocitrate dehydrogenase 1, the enzyme responsible for the decarboxylation of isocitrate to α-KG, are very common in human brain tumors. These mutations can decrease α-KG concentrations in glioma, which induces activation of oncogenic HIF pathways (Zhao S, Lin Y, Xu W, Jiang W, Zha Z, Wang P, Yu W, Li Z, Gong L, Peng Y, Ding J, Lei Q, Guan KL, Xiong Y. Glioma-derived mutations in IDH1 dominantly inhibit IDH1 catalytic activity and induce HIF-1alpha. Science 2009;324:261-265.).

Gallagher et al. (Detection of Tumor Glutamate Metabolism In Vivo Using 13C Magnetic Resonance Spectroscopy and Hyperpolarized [1-13C]glutamate. Ferdia A. Gallagher, Mikko I. Kettunen, Sam E. Day, De-en Hu, Magnus Karlsson, Anna Gisselsson, Mathilde H. Lerche, and Kevin M. Brindle. Magnetic Resonance in Medicine 66:18-23; 2011) have shown that [1-$^{13}$C]glutamate can be hyperpolarized and that the formation of α-KG can be demonstrated both in vitro and in vivo. This provides a first step to imaging these metabolites in vivo using DNP and demonstrates a new way in which the tumor levels of α-KG could be probed noninvasively. In this work, the Authors discuss a list of substrates and they highlight alanine transferase as the most relevant one. However, the Authors still pose challenges to be overcome, such as to find disease models which rapidly transport glutamate across the membrane, tissues which have a high enough enzyme activity to allow rapid label exchange, as well as cells which have a significant intracellular pool of α-KG.

In the work of Chaumeil et al. (Non-invasive assessment of IDH status in glioblastomas using dynamic 13C MRS of hyperpolarized α-ketoglutarate; Myriam Marianne Chaumeil, Sarah Woods, Robert M Danforth, Hikari Yoshihara, Alessia Lodi, Aaron Robinson, Joanna J. Philips, Sabrina M Ronen; Proc. Intl. Soc. Mag. Reson. Med. 20 (2012)) the hyperpolarization of α-KG has been used to monitor the status of the isocitrate dehydrogenase (IDH) enzyme, which mutations have been associated with gliomas and glioblastomas and with better prognosis, by detecting the formation of HP 2-hydroxyglutarate (2-HG). In both wild type and mutant cell lysates and also in live perfused wild-type cells, production of HP glutamate could be detected following injection of HP α-KG. No estimation of the HP glutamate formation is provided. This work is exclusively dedicated to IDH specific mutation, which is characteristic of a particular type of brain tumor.

Since the production of hyperpolarized metabolites which is suitable as an *in vivo* imaging agent is not without challenges, there is a need of alternative hyperpolarized imaging agents which can be used to obtain information about

metabolic activity, especially in the field of oncology. In particular, there is the need of correlating said metabolic information with the diagnosis and/or the efficacy of cancer therapy, in order to provide a non-invasive tool of imaging for diagnostic and monitoring purposes.

Still another problem is to find a way to provide an efficient tool for establishing the efficacy of a therapeutic treatment. This is even more urgent for certain diseases, such as tumors, where the response to the therapy may depend on individual variations of the response by the patient and the subsequent need to verify the efficacy of the therapy and adjust it.

A further problem, related to the previous one, is to distinguish particularly aggressive forms of tumors, preferably in early diagnosis stage, in order to adopt the proper therapeutic attack.

There remains the constant need to distinguish tumor area from surrounding healthy tissue in order to provide the physician, in particular the surgeon, with a field of intervention the most defined as possible. This could be achieved with more accurate and sensitive instrumental diagnostic tools.

## SUMMARY OF THE INVENTION

It has now been found that the conversion of hyperpolarized 1-$^{13}$C-2-oxoglutarate to hyperpolarized 1-$^{13}$C-glutamate gives rise to signals in cancer tissue different than in normal tissue. Therefore, said difference between the signals in tumor and non-tumor tissues of said hyperpolarized 1-$^{13}$C-glutamate signal can provide useful information for cancer diagnosis.

Furthermore, said hyperpolarized 1-$^{13}$C-glutamate signal can also be used for evaluating the efficacy of an anti-cancer therapy and/or to provide indications about time evolution of a tumor.

According to an aspect of the invention, the above oxoglutarate/glutamate conversion is preferably catalysed by aspartate transaminase (AST) enzyme.

An aspect of the present invention is a method of *in vivo* $^{13}$C-MR detection using an imaging medium comprising hyperpolarized 1-$^{13}$C-2-oxoglutarate, wherein signals of 1-$^{13}$C-glutamate are detected.

Preferably, in said method of $^{13}$C-MR detection said 1-$^{13}$C-2-oxoglutarate is metabolically converted into 1-$^{13}$C-glutamate through a reaction catalyzed by aspartate transaminase.

In an embodiment of the invention a first signal obtained from a region of interest is compared with a second signal (typically a signal derived from the subject. e.g. a signal obtained from a corresponding non-tumor/healthy tissue); said comparison is useful to determine a difference between tumor and non-tumor tissue, and more in particular can be used to provide a localization of a tumor. Furthermore, when a first signal obtained from a region of interest comprising a tumor tissue is compared with a second signal obtained from the same region of interest at an earlier time, the comparison between said first and said second signal can provide information about the grade of aggressiveness of the tumor and/or the efficacy of a therapy when treating said tumor by (immune)pharmacological and/or surgical and/or radio therapy.

In the above method of $^{13}$C-MR detection said signals can be used to generate a metabolic profile, based on the metabolic conversion of 1-$^{13}$C-2-oxoglutarate into 1-$^{13}$C-glutamate. Generating a metabolic profile is useful in detecting or providing indication of a pathological condition or a disease linked to said profile. In the present invention, said disease is a tumor. In an embodiment, said metabolic profile is determined in a region of interest (where the presence of a tumor tissue is known or suspected) and compared with a metabolic profile of reference (e.g. relative to a corresponding non-tumor tissue, typically a healthy tissue in the close proximity of the tumor tissue).

Another aspect of the invention is the above method further comprising the steps of:

d. calculating a difference between said first signal and second signal;
e. comparing said difference of step d) with a reference value, to produce a deviation value,
f. comparing the deviation value with a predetermined value.

A further aspect of the present invention is the above method, wherein said second signal is determined on a non-tumor tissue, further comprising the step of
g. providing an indication of possible tumor affection in case the deviation value is in absolute value higher than said predetermined value.

A further aspect of the present invention is the above method for operating an MRI system comprising steps a to f, wherein said second signal is determined in the region of interest at an earlier moment in time with respect to the first signal, and optionally stored in the system, said method further comprising the step of:
g'. providing an indication of tumor variation in case the deviation is in absolute value higher than said predetermined value.

A further aspect of the present invention is the above method for operating an MRI system comprising steps a to f, wherein said subject has undergone an anti-tumor treatment and wherein said second signal is determined in the region of interest, or on said second sample comprising said tumor-bearing or suspected tumor-bearing tissue, at an

earlier moment in time with respect to said first signal, and optionally stored in the system, said method further comprising the step of:

g". providing an indication of efficacy of said treatment if this deviation is in absolute value higher than a predetermined value.

In a preferred embodiment, said second signal is determined shortly before (typically within few days, e.g. 1 to 5), shortly after (typically within few days, e.g. 1 to 5) or at the beginning of the treatment.

The present invention provides the advantages of making available an imaging medium comprising hyperpolarized 1-$^{13}$C-2-oxoglutarate, which can be used in MRI technique for the diagnosis of tumors with a selective grade of distinction between tumor and non-tumor tissue.

A further advantage is represented by the possibility of taking different registrations of the MR signals of 1-$^{13}$C-glutamate in a tumor tissue, while an antitumor therapy is administered and to monitor the progress of the therapy.

A further advantage is represented by the possibility of detecting aggressive forms of tumors by monitoring the development of the formation of 1-$^{13}$C-glutamate in a tumor tissue.

These and other aspects and advantages of the present invention will be now disclosed in detail in the following description even by means of Figures and Examples.

FIGURES

Figure 1: Aspartate transaminase conversion of 1-$^{13}$C-2-oxoglutarate to 1-$^{13}$C-glutamate with no co-substrate, aspartate or glutamate as co-substrate (% of substrate, arbitrary units).

Figure 2: Uptake and conversion of 2-oxoglutarate using aspartate as co-substrate to produce glutamate in normal (THLE-3) and cancerous (HEP-G2) liver cells as well as in normal (PNT-1A) and cancerous (PC3) prostate cells. The numbers are given in nmol glutamate/min/million cells.

Figure 3: Conversion of hyperpolarized 1-$^{13}$C-2-oxoglutarate to hyperpolarized 1-$^{13}$C-glutamate in human prostate cancer cells.

Figure 4: Conversion of hyperpolarized 1-$^{13}$C-2-oxoglutarate to hyperpolarized 1-$^{13}$C-glutamate in Morris hepatocellular carcinoma bearing rat.

Figure 5: 1-$^{13}$C-glutamate peak in Morris hepatocellular carcinoma bearing rat.

Figure 6: Decay of hyperpolarized 1-$^{13}$C-2-oxoglutarate and hyperpolarized 1-$^{13}$C-glutamate in healthy rat liver.

Figure 7: 1-$^{13}$C-glutamate peak in healthy rat liver.

Figure 8: Intensity of the 1-$^{13}$C-glutamate peak after administration of 1-$^{13}$C-2-oxoglutarate pre and post treatment with Etoposide in EL-4 xenograft mouse lymphoma model (average over 4 mice).

Figure 9: Uptake and conversion of 2-oxoglutarate in prostate cancer (PC-3) treated cells compared to the untreated cancer cells.

Figure 10: Uptake and conversion of 2-oxoglutarate in liver cancer (Morris7777) treated cells compared to the untreated cancer cells (control).

Figure 11: Left: DNP conversion of hyperpolarized 1-$^{13}$C-2-oxoglutarate in prostate cancer (PC-3) cells. Two different treatments are compared to the untreated control cells: MS-275 and resveratrol. Right: Build-up curve of the hyperpolarized 1-$^{13}$C-glutamate metabolite from the resveratrol treated cells.

Figure 12: Left: DNP conversion of hyperpolarized 1-$^{13}$C-2-oxoglutarate in liver cancer (Morris7777) cells. Treatment with two different drugs; etoposide and sorafenib are compared to the untreated control cells. Right: Build-up curve of the hyperpolarized 1-$^{13}$C-glutamate metabolite from the etoposide treated cells.

Figure 13: Comparison between produced Hyperpolarized 1-$^{13}$C-lactate and Hyperpolarized 1-$^{13}$C-glutamate in liver cancer cells (Morris7777).

DETAILED DESCRIPTION OF THE INVENTION

Within the scope of the present invention, the term MRI means Imaging (typically for diagnostic purposes) by means of Magnetic Resonance (MR) as commonly intended in the state of the art and for example disclosed in WO200977575 and the references cited therein.

Within the scope of the present invention, the "imaging medium" and "contrast agent" are used synonymously as commonly intended in the state of the art and for example disclosed in WO200977575 and the references cited therein.

Within the scope of the present invention, the terms "hyperpolarization", "hyperpolarized" or similar mean enhancing the nuclear polarization of NMR active nuclei present in the high $T_1$ agent as commonly intended in the state of the art and for example disclosed in WO200977575 and the references cited therein.

Within the scope of the present invention, the term Dynamic Nuclear Polarization (DNP) is a technique in

Magnetic Resonance Imaging as commonly intended in the state of the art and for example disclosed in WO200977575 and the references cited therein.

Within the scope of the present invention, the term "hyperpolarized" means the nuclear spin polarization of a compound higher than thermal equilibrium.

Within the scope of the present invention "MRI system" means apparatus, equipment and all features and accessories useful for performing MR experiments, in particular for diagnostic purposes.

The hyperpolarized 2-oxoglutarate is prepared by Dynamic Nuclear Polarization (DNP), which is a known method disclosed, for example, in WO9935508, and in particular in WO2011124672.

In a preferred embodiment, the method for the hyperpolarization of molecules comprises the use of a stable hyperpolarized precursor which can be readily transformed into the desired hyperpolarized substrate upon dissolution in an aqueous carrier. Said method is performed according to WO2011124672.

In an embodiment of the present invention, within the frame of the disclosure of the above mentioned WO2011124672, said precursor is hydrolysable. Preferred precursors are selected from organic cyclic or linear anhydrides, symmetric or mixed; cyclic or acyclic diketenes, esters, lactones or amides. More preferred precursors are mono-ethyl or diethyl ester.

With the term "hydrolysable precursor" it is herein intended that when said precursor is in a suitable environment, for example before or upon administration to a subject, it is converted into 2-oxoglutaric acid, or its anionic form 2-oxoglutarate, through a hydrolysis reaction. Preferably the precursor is hydrolysed by dissolving the hyperpolarized precursor in a suitable aqueous solvent before administration or addition thereof. Preferably at least 50% of the precursor is hydrolyzed, more preferably at least 75% and even more preferably at least 90%, particularly preferred being a transformation of at least 95% of the precursor into 2-oxoglutaric acid, or its anionic form 2-oxoglutarate. Optionally the hydrolysis of the precursor is catalyzed by a hydrolyse enzyme, as described e.g. in WO2011124672.

Hydrolysable precursors of 2-oxoglutarate for use in the method of the invention comprise organic compounds which upon dissolution in an aqueous solvent are transformed into the desired 2-oxoglutarate (or its undissociated form oxoglutaric acid), alone or in admixture with one or more by-products, the latter preferably being pharmaceutical acceptable (depending on the type of precursor, these by-products may comprise for instance other carboxylic acids, alcohols, amides etc.). Examples of suitable precursors include, for instance, anhydrides (cyclic or linear, symmetric or mixed); ketenes (mono- or di-ketene); esters (mono- or di-ester, linear or cyclic) or amides (mono- or di-amide, linear or cyclics). Preferred precursors are esters, anhydrides or amides of 2-oxoglutarate, particularly preferred being esters and more in particular diethyl ester or mono-ethyl ester of 2-oxoglutarate. Most preferred is mono-ethyl ester of 2-oxoglutarate. These precursors are cited as being part of the present disclosure but they are not part of the claimed invention.

Essentially, the method of operating an MRI system used for the present invention comprises the steps of a) recording an MR signal from said excited nuclei, preferably from the excited $^{13}C$ nuclei of said hyperpolarized 1-$^{13}C$-glutamate.; and b) comparing a first MR signal deriving from said tumor with a second MR signal deriving from said subject or from a sample thereof.

In an embodiment of the invention, said first signal deriving from said tumor is higher than said second MR signal.

In an alternative embodiment of the invention, said first signal deriving from said tumor is lower than said second MR signal.

In an embodiment of the present invention, as shown in steps c) to f) above, the MRI system can process said first signal and said second signal by comparing each other, calculating a difference between the two signals and comparing said difference with a reference value; as shown in step g above, if this comparison provides a value which is, in absolute value, higher than a predetermined value, then said MRI system provides an indication of possible tumor affection.

An MRI system adapted to perform a method according to the present invention is also an aspect of the present disclosure. In particular, said MRI system possesses all the features required for performing recording of MR signals and comparison of said signals as provided by the method of the invention.

In a preferred embodiment, said MRI system is adapted to record an MR signal from the excited nuclei of hyperpolarized 1-$^{13}C$-glutamate. The use of said system for monitoring the response of a subject affected by a tumor to antitumor therapy (step g') or for evaluating the aggressiveness of a tumor (step g") are further aspects of the present invention.

The active uptake of the hyperpolarized 1-$^{13}C$-2-oxoglutarate by the cancer cells, when said hyperpolarized 1-$^{13}C$-2-oxoglutarate is administered to a patient, is often the limiting factor in producing the hyperpolarized 1-$^{13}C$-glutamate signal.

Antineoplastic drugs prevent cancer cells from proliferate and eventually kill the cells. Said killed cells liberate several enzymes, such as aspartate transaminase which converts 2-oxoglutarate to glutamate. The cell liberation of said enzyme results in a faster conversion of and a higher signal of hyperpolarized 1-$^{13}C$-glutamate, since the above mentioned limiting factor of the active uptake of 1-$^{13}C$-2-oxoglutarate is eliminated.

Therefore, these types of therapies can be monitored by the administration of hyperpolarized 1-$^{13}C$-2-oxoglu-

tarate, which in a higher degree is converted to hyperpolarized 1-$^{13}$C-glutamate as a consequence of the death of cancer cells induced by the therapy.

In several cancer cell types transaminases (in particular aspartate transaminase) are highly expressed and the enzyme activities are high due to elevated amino acid concentrations, which usually includes aspartate.

According to an embodiment of the present invention, it is possible to increase the cellular aspartate or glutamate concentration by pre- or co-administrating unlabelled, non-hyperpolarized aspartate or glutamate.

In the methods of the present invention, unlabelled, non-hyperpolarized aspartate or glutamate will be administered to the subject positioned in the MR system.

Administration of aspartate or glutamate can be effected by any method of administration and can be made immediately before, immediately after the treatment with hyperpolarized 1-$^{13}$C-2-oxoglutarate. Aspartate or glutamate can also be co-administered with the hyperpolarized 1-$^{13}$C-2-oxoglutarate, for example in the same administration device.

According to the present invention, hyperpolarized 1-$^{13}$C-2-oxoglutarate can be exploited as a marker of targeted therapies, where for targeted therapy is intended the targeting of molecules important for the carcinogenesis of the cancer cells.

Antineoplastic drugs work through different mechanisms but their activity is usually reflected in general changes of the activities of housekeeping enzymes, such as aspartate transaminase. Therefore, a broad range of therapies with antineoplastic drugs can be monitored by the method of the present invention.

Generally, the present invention is applicable to aspartate transaminase overexpressing tumors.

Examples of said tumors are tumors selected from the group consisting of prostate, breast, liver, colon, lymphoma and ovarian tumors.

Nevertheless, the method of the present invention is applicable also to those tumors where the expression of aspartate transaminase is lower than the one in non-tumor tissues, since the deviation from the reference value is expressed as absolute value.

Preferred therapies are those with highly efficient cell killing antineoplastic drugs, such as antimetabolites, DNA replication inhibitors, histone deacetylase inhibitors and hormonal antineoplastic drugs.

Hyperpolarized 1-$^{13}$C-2-oxoglutarate may also be a marker of the effect of phytochemicals with anti-tumor efficacy.

Particularly preferred therapies are the ones in which one of the following antineoplastic drugs is used: etoposide (DNA replication inhibitor), gemcitabine (antimetabolite), MS-275 (Entinostat) (Histone Deacetylase Inhibitor), sorafenib (targeted therapy), Resveratrol and SNF (sulforaphane) (phytochemicals).

In carrying out the methods of the present invention, the first signal ($S_1$), the second signal ($S_2$) and the reference value (R), depend on how the methods of the invention are applied.

Typically, in order to have comparable data, the MR signals obtained in the method of the invention are normalized with respect to the corresponding signal of 1-$^{13}$C-2-oxoglutarate.

In case the purpose of the method is to gather clinical data for a diagnosis of tumor, said first signal $S_1$ is the $^{13}$C-glutamate signal detected in the region of interest comprising the alleged tumor, while the second signal $S_2$ is the one produced by non-tumor tissue; the reference value R is either equal to $S_2$ or, in case no $^{13}$C-glutamate signal is detected in the healthy tissue under consideration, R is set to 3 times the noise standard deviation. Preferably, non-tumor tissue is surrounding the tumor, so that the MR system can provide an accurate imaging of the tumor, which is of great importance for the evaluation of surgical intervention.

In case the purpose of the method is a follow-up of antitumor therapy, said second signal and said reference value correspond to the $^{13}$C glutamate signal detected in the tumor before, or at the start or at a certain point after the beginning of therapy and said second signal is the one produced by the same tumor after a certain period subsequent to the detection of said first signal.

In case the purpose of the method is to determine aggressiveness of a tumor, said first signal is the one produced by the tumor at the start of the determination, and said second signal is the one produced by the same tumor after a certain period subsequent to the detection of said first signal. Again the reference value is set equal to the first signal.

The first and second MR signals can be obtained either as single signals or calculated as a mean value of a plurality of respective signals determined (from different voxels) in a selected region of interest ($S_1$) or in a non-tumor tissue ($S_2$).

In an embodiment of the invention, said first signal and said second signal can be directly compared, either as single signals or as mean values of a plurality of signals, to obtain the desired information on the tumor tissue. In an alternative embodiment of the invention, the signals can be used to generate a parametric image and the comparison can be performed by comparing the zones of said image corresponding to the first and said second signal.

According to the present invention, a difference between said first and said second signal is calculated.

In this context, with the term "calculation" it is intended that a calculation leading to the obtainment of a numerical result is performed. The calculated difference ($S_1$-$S_2$) is important for the different scopes of the present invention.

This difference is compared with the reference value to produce a value representing the deviation (D) of said

difference from said reference value:

$$D = (S_1 - S_2)/R.$$

If it is determined that this deviation provides a value which is, in absolute value, higher than a predetermined value, this deviation provides an indication of possible tumor affection, of the efficacy of the antitumor therapy or of tumor aggressiveness, depending on the purpose of the method of the invention.

For instance, in an embodiment of the invention, said predetermined value can be set at 2; accordingly, if the calculated value "D" is equal or higher than 2, this can be indicative of a possible presence of a tumor in the region of interest, of the efficacy of the antitumor therapy or of tumor aggressiveness, depending on the purpose of the method of the invention. Preferably a deviation value D of from 2 to 10 can be indicative of said presence, efficacy or aggressiveness, more preferably a deviation from 2 to 20, even more preferably a deviation from 2 to 40, particularly preferred is a deviation from 2 to 60, maximally preferred is a deviation from 2 to 80, the most preferred is a deviation from 2 to 100 or higher.

In an embodiment of the invention, the method is performed on a subject who is suspected to suffer or suffers from a tumor.

In another embodiment of the present invention, the above method is performed on a subject who is undergoing or has been subjected to an antitumor treatment and the reference value is the signal of $^{13}C$ glutamate in said region of interest determined before, during or after said treatment. As above, if a deviation D is calculated which is higher, in absolute value, than a predetermined value (e.g. higher than 2, and preferably within the above indicated ranges), this provides an indication of the efficacy of the antitumor treatment.

In some embodiments, the present invention can be used in the field of so-called "personalized medicine", or similarly intended. As explained above, tumor therapy is affected by variations in its efficacy even on the same type of tumor and with the same anticancer therapeutic protocol. Such variations are due to the different individual responses by the patients to a therapy.

Carrying out the method of the present invention allows to monitor (follow-up) the efficacy of a tumor therapy and, in case, providing the physician with useful information for adapting the therapy to the patient.

In cell types where glutamate concentrations are high and one or more transaminase enzymes are highly expressed, the conversion of hyperpolarized 1-$^{13}C$-2-oxoglutarate to hyperpolarized 1-$^{13}C$-glutamate will give rise to a higher signal than in cells where the concentration of glutamate is lower and the expression and/or activity of one or more transaminase enzymes are lower.

Glutamate concentrations are generally higher in many cancer types compared to the corresponding normal cells in the surrounding tissue.

In particular, cancers for which the invention is preferably applicable are prostate, breast, liver, colon, lymphoma and ovarian cancers.

In these tumors, different transaminase enzymes are highly expressed. In particular, the inventors of the present invention have found that aspartate transaminase is more highly expressed; therefore, said enzyme is an important enzyme to target in tumors. Aspartate transaminase (AST) catalyzes the reversible transfer of an $\alpha$-amino group between aspartate and glutamate allowing the interconversion of aspartate and $\alpha$-oxoglutarate to oxaloacetate and glutamate:

*Aspartate (Asp) + $\alpha$-oxoglutarate $\leftrightarrow$ oxaloacetate + glutamate (Glu)*

It has been found that in tumors, in particular, but not exclusively, in the tumors listed above, aspartate is the preferred amino acid partner which allows a better conversion of 2-oxoglutarate into glutamate, therefore aspartate transaminase is the enzyme which is more highly expressed in said tumors. This finding is unexpected in view of the prior art, especially in view of the above mentioned Gallagher et al., focusing on alanine transferase as a hallmark for cancer.

Advantageously, the metabolic marker hyperpolarized 1-$^{13}C$-2-oxoglutarate has a longer T1 than hyperpolarized $^{13}C$-glutamate and therefore is a better hyperpolarized metabolic marker. Although hyperpolarized 1-$^{13}C$-2-oxoglutarate is the substrate of many transaminases (belonging to the family EC 2.6.1.x), we have found that aspartate is the best amino acid partner and therefore that aspartate transaminase (EC 2.6.1.1) is the most important enzyme to target with hyperpolarized 1-$^{13}C$-2-oxoglutarate in tumors .

In cancer cell types where aspartate transaminase is highly expressed, an elevation of cellular aspartate concentrations will result in elevated hyperpolarized 1-$^{13}C$-glutamate signal when hyperpolarized 1-$^{13}C$-2-oxoglutarate is administered as a substrate. Therefore, tumors with a high cellular expression of aspartate represent a preferred embodiment of the present invention.

The activity of the aspartate transaminase in said cancer cells is high due to elevated amino acid concentrations

with affinity for this enzyme, which usually includes aspartate, and it can be increased by pre or co-administrating unlabelled, non-hyperpolarized aspartate.

In an embodiment of the present invention, unlabelled, non-hyperpolarized aspartate or glutamate is administered together with the 1-$^{13}$C-2-oxoglutaric acid as a co-substrate, in case there is not enough aspartate or glutamate available for the cells.

If the tumor is particularly aggressive, cells grow faster than angiogenesis resulting in large areas of necrosis within the tumor itself. In this case, the presence of many necrotic cells will give rise to an even higher variation of the hyperpolarized 1-$^{13}$C-glutamate signal. Therefore, in an embodiment of the present invention, if the deviation D is higher, in absolute value, than a predetermined value (e.g. higher than 2, and preferably within the above indicated ranges) this provides an indication of the progression rate of the cancer and thus of the possible presence of an aggressive cancer.

Many of the transaminases which are more highly expressed in tumors, such as aspartate transaminase, have cytosolic isoforms. When a cancer undergoes anticancer therapy, in the case of a cytotoxic therapy cancer cells die and become necrotic. In cell necrosis, said enzymes, as well as various amino acids, leak to the intercellular space. The necrosis thereby makes it possible to have a more direct access to these high activity enzymes since the limitations of transport across the cell membranes can be circumvented. Therefore, more of the administered hyperpolarized 1-$^{13}$C-2-oxoglutarate is converted to hyperpolarized 1-$^{13}$C-glutamate and correspondingly a different rate of signal from hyperpolarized 1-$^{13}$C-glutamate is obtained.

2-oxoglutarate crosses the cell membrane and the present invention is able to image the cellular difference between healthy and cancerous cells in viable cells due to differences in intracellular hyperpolarized 1-$^{13}$C-glutamate signal. These differences may have different origin (different uptake rates of the substrate, hyperpolarized 1-$^{13}$C-2-oxoglutarate, or differences in intracellular glutamate concentrations or differences in enzyme activities most dominantly differences in activities of aspartate transaminases or differences in the expression (concentration) of the relevant enzymes). This is an advantage of the present invention, allowing to obtain better images of tumors.

In the embodiment of the present invention referred to therapy follow-up, a change in hyperpolarized 1-$^{13}$C-glutamate signal may be due to different reasons. In fact, in the presence of a cytotoxic therapy, cancer cells typically die (become necrotic) thus possibly causing an extracellular leakage of the enzyme(s) that convert hyperpolarized 1-$^{13}$C-2-oxoglutarate into 1-$^{13}$C-glutamate. Leakage of the enzyme(s) will change the rate of the enzymatic reaction and the amount of 1-$^{13}$C-glutamate relative to the intracellular 1-$^{13}$C-glutamate signal obtained in the non-treated cancer cells, due to the elimination of the often rate-limiting step of taking up the substrate hyperpolarized 1-$^{13}$C-2-oxoglutarate.

Even in therapies where necrosis cell death is less common, a change in the hyperpolarized 1-$^{13}$C-glutamate signal can be detected, probably due to changes in expression or activity of a transaminase enzyme (e.g. aspartate transaminase) or a change in the cellular uptake of 2-oxoglutarate.

Therefore, a variation of the hyperpolarized 1-$^{13}$C-glutamate signal, when compared with the signal obtained before the anticancer therapy, may provide an indication of the efficacy of the therapy and can thus be used as information for monitoring the therapy itself.

The present method thus provides the physician with useful information about possible tumor affection, efficacy of an antitumor therapy or tumor aggressiveness, which may be used, together with other data and/or information, such as hematic parameters or imaging data in the assessment of a pathological (in particular tumor) condition, of the efficacy of an anti-tumor therapy or of the progression rate of a tumor.

Typical metabolic imaging procedures with 1-$^{13}$C-2-oxoglutarate in human subjects should be performed at magnetic fields $\geq$ 1 T. Field strengths of 3 T or higher are preferred since the spectral separation between the injected substrate (1-$^{13}$C-2-oxoglutarate) and the observed metabolite (1-$^{13}$C-glutamate) scales linearly with the intensity of the applied field. The MR scanner should be capable to detect $^{13}$C signals in addition to 1H and although not always mandatory, surface or endoscopic radiofrequency coils could allows to achieve better results in specific organs. For prostate investigation for instance, an endorectal $^{13}$C is expected to strongly increase the sensitivity of the method with respect to a standard whole body resonator. Being the hyperpolarized signals typically available for a time range in the order of 3 to 5 times the longitudinal relaxation rate of 1-$^{13}$C-2-oxoglutarate, the total acquisition time for a metabolic MR procedure will not exceed 3 min. Spectroscopic imaging sequences such as Single Voxel Spectroscopy (SVS) or Chemical Shift Imaging (CSI) need to be used in order to separate the signal coming from the substrate from that coming from the metabolic product. Fast spectroscopic imaging sequences such as EPSI are preferred due to the limited time available for the acquisition.

In order for the method to provide enough sensitivity, 1-$^{13}$C-2-oxoglutarate formulations and dissolution/transport protocols which allow maintaining at least 10% polarization at time of injection are preferred, in particular for in vivo applications. Preferably, at least of about 20% polarization is maintained, more preferably at least of about 30% polarization is maintained, even more preferably at least of about 60% polarization is maintained, most preferably at least of about 80% polarization is maintained.

The present invention will be further illustrated by the following examples.

## EXAMPLES

All the chemicals and reagents are commercially available or can be prepared according to well-known methods of the art.

### Example 1: DNP preparation and dissolution of 2-oxoglutaric-(1)-ethyl ester

2-oxoglutaric-(1)-ethyl ester (31 mg, 0.18 mmol) was added to an Eppendorf tube and mixed with 0.7 mg (0. 45 μmol) of the carboxylic acid form of the finland radical (tris{8-carboxyl-2,2,6,6-tetramethyl-benzo(1,2-d:4,5-dS)bis(1,3)dithiole-4-yl}methyl, carboxylic acid form). This preparation was 6.5 M with respect to 2-oxoglutarate. The ethyl ester formed a glass upon rapid freezing. 31 mg of this composition was transferred from the Eppendorf tube to a sample cup and the sample cup was inserted into a DNP polarizer. The composition was hyperpolarized under DNP conditions at 1.2 K in a 3.35 T magnetic field under irradiation with microwave (93.900 GHz). The sample was hyperpolarized for 90 min.

The sample was dissolved in 6 ml $D_2O$ with added NaOH (35 μl of 10 M). The solution was collected directly into a 10 mm NMR tube and transferred to a 14.1 T magnet (pH 11) where a time series of 5 degree 1D $^{13}C$-NMR spectra were recorded with a total delay between the pulses of 3 s. The ester was hydrolyzed up to at least 95 %, after 15 s. The 2-oxoglutaric-(1)-ethyl ester was easily hydrolyzed to 2-oxoglutarate during the time of the experiment.

### Example 2: DNP preparation and dissolution of 2-oxoglutaric acid

The carboxylic acid form of the Ox063 radical (tris(8-carboxy-2,2,6,6-(tetra(hydroxyethyl)-benzo-[1,2-4,5'] -bis-(1,3)-dithiole-4-yl)-methyl) (2.6 mg, 1.9 μmol) was dissolved in DMSO (63 μl, 69.9 mg). 1-$^{13}C$-2-oxoglutaric acid (35.5 mg, 0.24 mmol) was dissolved (sonication and whirling) in 23 μl (26.6 mg) of the DMSO radical solution. To the solution was added Gadobop (1.1 mg of 100 μmol / g solution in DMSO).

A sample (62.8 mg, 0.14 mmol) of this composition was transferred from the Eppendorf tube to a sample cup and the sample cup was inserted into a DNP polarizer. The composition was hyperpolarized under DNP conditions at 1.2 K in a 3.35 T magnetic field under irradiation with microwave (93.900 GHz). The sample was hyperpolarized for 90 min.

The sample was dissolved in 5 ml phosphate buffer (40 mM, pH 7.3) with addition of 12M NaOH (44 μl). The solution was collected directly into a 10 mm NMR tube and transferred to a 14.1 T magnet (pH 7.2) where a time series of 5 degree 1D $^{13}C$-NMR spectra were recorded with a total delay between the pulses of 3 s. The polarization was 14 % (30 s after dissolution) and the liquid state T1 was 27 s at 14.1 T and 37 C.

### Example 3: Importance of co-substrate in the conversion of Hyperpolarized 1-$^{13}C$-2-oxoglutarate to 1-$^{13}C$-glutamate in a transaminase reaction.

12.1 mg, 47 μmol of a 1-$^{13}C$-2-oxoglutaric acid sample made according to example 2 was hyperpolarized. The sample was dissolved in 5ml phosphate buffer (40 mM, pH 7.3) with addition of NaOH (8 μl, 12 M). The pH after dissolution was 7.3. From this hyperpolarized solution aliquots of 300 μl were taken and added one at a time to 5 mm NMR tubes (1-3) kept at 37° C containing the following:

Tube 1: 2.5 μl (3.2 U) aspartate transaminase + 100 μl (120 mM) glutamate + 200 μl 40 mM phosphate buffer pH.7.3.
Tube 2: 2.5 μl (3.2 U) aspartate transaminase + 100 μl (120 mM) aspartate + 200 μl 40 mM phosphate buffer pH.7.3.
Tube 3: 2.5 μl (3.2 U) aspartate transaminase + 300 μl (40 mM) phosphate buffer pH.7.3.

Immediately following the addition of hyperpolarized substrate into the prewarmed tube 1 the sample was mixed by turning twice and inserted into a 14.1 T magnet. A low pulse angle experiment was performed (15 degree pulses every 0,5 s for 12 s). Following the end of the experiment the procedure was repeated for tube 2 and tube 3.

If no co-substrate is present (tube 3) then no product can be formed since the enzyme needs a nitrogen donor to make glutamate from 2-oxoglutarate. This can be appreciated in the reaction scheme shown below. Although the enzyme aspartate transaminase uses aspartate as the co-substrate it is clearly possible to use glutamate as the co-substrate and product at the same time. At the conditions used in this experiment the two amino acids are almost equally effective, Figure 1.

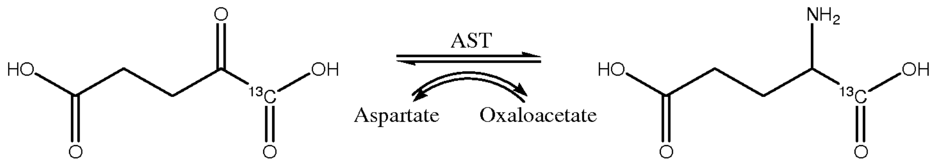


1-$^{13}$C-2-Oxoglutarate 1-$^{13}$C Glutamate

Reaction scheme of aspartate transaminase reaction.

**Example 4: Conversion of 2-oxoglutarate in healthy and cancerous cells as measured with conventional biochemical method.**

A combination of cellular uptake of 2-oxoglutarate and conversion of 2-oxoglutarate to glutamate was measured using a UV assay. Following incubation of the cells with 2-oxoglutarate for 30 minutes at 37 °C the cells were disrupted and a perchloric acid extract was made to precipitate the enzymes, whereafter the concentration of the formed product, glutamate, was measured.

The following UV assay for measuring glutamate production was applied.

The assay consists of two parts, as shown in the reaction scheme below. In the first part, glutamate is generated by incubating 0.5 million (0.5*10$^6$)viable cells with 20 mM 2-oxoglutarate and 20 mM aspartate for 30 minutes at 37 °C. In the second part the glutamate concentration is measured using commercially available GDH (glutamate dehydrogenase) enzyme to generate 2-oxoglutarate and NADH. The latter is an unfavorable reaction and the reaction is therefore forced in this direction by trapping of the produced NADH in the INT (Iodonitrotetrazolium) reaction.

1a)

$$\text{2-Oxoglutarate + Amino acid} \xrightarrow{\text{Transaminases}} \text{Glutamate + Keto acid}$$

1b)

$$\text{2-oxoglutarate} + NH_3 + \text{NADH} \xrightarrow{GDH_{cell}} \text{Glutamate} + H_2O + NAD^+$$

2)

$$\text{Glutamate} + H_2O + NAD^+ \xrightarrow{GDH_{iso}} \text{2-Oxoglutarate} + NH_4^+ + \text{NADH}$$

3)

$$\text{NADH + INT} \rightarrow NAD^+ + \text{INT-formazan}$$

Reaction scheme for the assay determining glutamate concentration from cells incubated with 2-oxoglutarate. GDHcell refer to the cellular presence of glutamate dehydrogenase which will convert 2-oxoglutarate to glutamate when 2-oxoglutarate is incubated with whole cells in the first part of the assay. GDHiso refer to the isolated and purified glutamate dehydrogenase enzyme which is added in excess in the second part of the assay.

Two cancer cell lines, a human prostate carcinoma (PC-3) and a human liver carcinoma (HEP-G2) were compared with immortalized human normal cells from prostate (PNT-1A) and from liver (THLE-3). A comparison of the conversion of 2-oxoglutarate to glutamate in these four cell lines are shown in Figure 2.

The conversion of 2-oxoglutarate in glutamate using aspartate as a co-substrate is higher in carcinoma cells than in normal cells from the same organ.

**Example 5: Conversion of Hyperpolarized 1-$^{13}$C-2-oxoglutarate to hyperpolarized 1-$^{13}$C-glutamate in human prostate cancer cells as measured with Dynamic Nuclear Polarization Magnetic Resonance.**

30 μmol of a 1-$^{13}$C-2-oxoglutaric acid sample made according to example 2 was hyperpolarized. The sample was dissolved in 5ml phosphate buffer (40 mM, pH 7.3) with addition of NaOH (5 μl, 12 M). The pH after dissolution was 7.3.

10 million prostate cancer cells (PC-3) were harvested and redissolved in 500 μl phosphate buffer (PBS). To this solution was added 100 μl of a 250 mM aspartate solution and the mixture was placed in a 10 mm NMR tube and placed with a connecting tubing in a 14.1 T magnet at 37° C.

2 ml of the dissolved hyperpolarized 1-$^{13}$C-2-oxoglutarate was injected through the connecting line (dead volume 1 ml) resulting in a total substrate concentration of 3.5 mM. A series of 30 degree pulses every 2 s (56 scans in total) was acquired. The acquisition was started just before injection of the hyperpolarized substrate.

A build-up of produced hyperpolarized 1-$^{13}$C-glutamate in prostate cancer cells can be seen in Figure 3. It can appreciated from figure 3 that hyperpolarized 1-$^{13}$C-2-oxoglutarate is taken up by PC-3 cells and converted into hyperpolarized 1-$^{13}$C-glutamate on the time scala of the DNP experiment. While the hyperpolarized substrate is decaying the product is building up due to conversion hyperpolarized 1-$^{13}$C-2-oxoglutarate and eventually decaying due to hyperpolarized signal decay (T1). In comparison to example 4 above, the amount of signal in liver cancer cells is expected to be higher and the amount of signal in healthy cells is expected to be lower.

**Examples 6-7: In vivo data illustrating value of hyperpolarized 1-$^{13}$C-2-oxoglutarate for diagnosing cancer**

Example 6: Conversion of hyperpolarized 1-$^{13}$C-2-oxoglutarate into hyperpolarized 1-$^{13}$C-glutamate in liver cancer in the living rat.

Morris hepatocellular carcinoma has been selected as test system. One million McA-RH7777 cells have been suspended in 0.2 mL of Dulbecco's Modified Eagle's Medium (DMEM) medium and injected under the hepatic capsula of the liver left lobe of anesthetized Buffalo rats (strain widely used in literature as a suitable model for oncological studies).

DNP experiment has been performed 3 weeks after cells inoculation. 0.24 mmol of a 1-$^{13}$C-2-oxoglutaric acid sample made according to example 2 was hyperpolarized. The sample was dissolved in 5 ml phosphate buffer (40 mM, pH 7.3) with addition of aspartate (48 mM) and NaOH (5 μl, 12 M). The pH after dissolution was 7.5.

2.5 ml of the dissolved hyperpolarized 1-$^{13}$C-2-oxoglutarate was injected intravenously at an injection rate of about 0.25 mL/s through a catheter placed in the tail vein of the animal resulting in a total administered dose of 0.4 mmol/kg and a substrate concentration of 48 mM. A series of 10 degree pulses every 3 s (64 scans in total) was acquired. Only the signal originating from the tumor mass was collected by means of slice selective pulses. The acquisition was started 15 s before injection of the hyperpolarized substrate.

The build-up of in vivo produced hyperpolarized 1-$^{13}$C-glutamate in liver cancer can be seen in Figure 4. It can be appreciated from Figure 4 that hyperpolarized 1-$^{13}$C-2-oxoglutarate is taken up by liver cancer cells and converted into hyperpolarized 1-$^{13}$C-glutamate on the time scale of the DNP experiment. While the hyperpolarized substrate is decaying the product is building up due to conversion hyperpolarized 1-$^{13}$C-2-oxoglutarate and eventually decaying due to hyperpolarized signal decay (T1). The total sum peak (over the whole temporal series) is shown in Figure 5.

Example 7: Conversion of hyperpolarized 1-$^{13}$C-2-oxoglutarate into hyperpolarized 1-$^{13}$C-glutamate in the normal liver of the living rat.

Buffalo rats have been selected as system model, in order to have a comparison between healthy and diseased tissue on the same strain.

DNP experiment has been performed as described in example 6.

No build-up of produced hyperpolarized 1-$^{13}$C-glutamate in healthy liver tissue can be observed as shown in Figure 6. It can be noticed that similarly to hyperpolarized 1-$^{13}$C-2-oxoglutarate, the signal originating from hyperpolarized 1-$^{13}$C-glutamate has a monotonic decay, due to T1 relaxation and no building up is identifiable. The signal has to be ascribed to the presence of an impurity rather than to in vivo metabolism. The total sum peak (over the whole temporal series) is shown in Figure 7.

**Example 8: In vivo data illustrating value of hyperpolarized 1-$^{13}$C-2-oxoglutarate for monitoring therapy.**

EL-4 xenograft mouse lymphoma model has been selected as test system to verify the ability of detecting tumour response to a DNA targeting therapy. Signals for hyperpolarized 1-$^{13}$C-glutamate (product) from hyperpolarized 1-$^{13}$C-2-oxoglutarate (substrate) in lymphoma mouse cancer pre versus post treatment with etoposide have been evaluated.

Five million EL-4 cells were suspended in 0.1 mL Roswell Park Memorial Institute (RPMI-1640) medium and subcutaneously injected in the flank of 7 weeks old female C57BL/6 mouse.
DNP experiment has been performed 9-10 days after cells inoculation pre Etoposide treatment and 10-11 days after cells inoculation post single dose Etoposide treatment (a dose of 2 mg/mouse has been i.p. administered by injection of 300 μL of Eposin 20 mg/mL diluted 1:3 in saline solution). DNP sample has been prepared as describes in example 6.
180 ul of the dissolved hyperpolarized 1-$^{13}$C-2-oxoglutarate was injected intravenously at an injection rate of about 2 mL/min through a catheter placed in the tail vein of the animal resulting in a total administered dose of 0.4 mmol/kg and a substrate concentration of 48 mM. Signal has been acquired as described in example 6.
The model shows that the signal of hyperpolarized 1-$^{13}$C-glutamate increases two times post therapy compared to pre therapy (Figure 8).

**Example 9: Conversion of 2-oxoglutarate in cancerous cells before and after treatment as measured with conventional biochemical method.**

The UV assay and conditions used in example 4 were applied. Prior to harvesting the cells have been treated with MS-275, resveratrol, etoposide, or Sorafenib, as follows:

PC-3 cells: MS-275 0.5 μM, for 48 hours; Resveratrol 50 μM for 48 hours.
Morris7777 cells: Etoposide 15 μM, for 24 hours; Sorafenib 0.5 μM for 48 hours.

The treatment protocols were evaluated with a standard cellular method, trypan blue staining which provide the total cell numbers and cell viability.
Uptake and conversion of 2-oxoglutarate were measured as described in example 4.
Results are shown in figures 9 (PC-3 cells) and 10 (Morris7777 cells).
In comparison to the untreated prostate cancer cells (control) the conversion of 2-oxoglutarate to glutamate in prostate cancer cells treated with resveratrol is higher whereas no change was seen with the MS-275 treatment. This result is supported by measured cell number and cell viability in the different treatments, Table 1. From table 1 it can be seen that the total cell number is significantly decreased in the resveratrol treated cells and also the viability has decreased, both indicative of an effective treatment.

*Table 1. Treatment effect on cell number and cell viability upon MS-275 and resveratrol treated PC-3 cells.*

| Cell treatment | Cell number | Cell viability |
|---|---|---|
| Untreated PC-3 cells (control) | 7.7 million | >97% |
| MS-275 treated PC-3 cells | 4.5 million | >97% |
| Resveratrol treated PC-3 cells | 1.7 million | >94% |

In comparison to the untreated liver cancer cells (control) the conversion of 2-oxoglutarate to glutamate in liver cancer cells treated with Etoposide is higher whereas no change was seen with the Sorafenib treatment. This result is supported by measured cell number and cell viability in the different treatments, Table 2. From table 2 it can be seen that the total cell number is significantly decreased in the Etoposide treated cells and also the viability has decreased significantly both indicative of an effective treatment.

*Table 2. Treatment effect on cell number and cell viability upon Etoposide and Sorafenib treated Morris7777 cells.*

| Cell treatment | Cell number | Cell viability |
|---|---|---|
| Untreated Morris7777 cells (control) | 8.3 million | >94% |
| Etoposide treated Morris7777 cells | 5.5 million | <85% |
| Sorafenib treated Morris7777cells | 8 million | >89% |

In both cases where the treatment protocols have worked the effect of the treatments can be diagnosed with a higher amount of produced glutamate from 2-oxoglutarate.

**Example 10: Conversion of Hyperpolarized 1-$^{13}$C-2-oxoglutarate to hyperpolarized 1-$^{13}$C-glutamate in treated and untreated cancerous cells as measured with Dynamic nuclear polarization magnetic resonance.**

30 μmol of a 1-$^{13}$C-2-oxoglutaric acid sample made according to example 2 was hyperpolarized. The sample was dissolved in 5ml phosphate buffer (40 mM, pH 7.3) with addition of NaOH (5 μl, 12 M). The pH after dissolution was 7.3.

10 million treated or untreated prostate cancer cells (PC-3) or 10 million treated or untreated liver cancer cells (Morris7777), were harvested and dissolved in 500 μl phosphate buffer (PBS). Treatment protocols were similar to those described in example 7. To cell suspension was added 100 μl of a 250 mM aspartate solution and the mixture was placed in a 10 mm NMR tube and placed with a connecting tubing in a 14.1 T magnet at 37°C.

2 ml of the dissolved hyperpolarized 1-13C-2-oxoglutarate were injected through the connecting line (dead volume 1 ml) resulting in a total substrate concentration of 3.5 mM. A series of 30 degree pulses every 2 s (56 scans in total) was acquired. The acquisition was started just before injection of the hyperpolarized substrate.

Results are shown in figures 11 and 12.

In both cases where the treatment protocols have worked the effect of the treatments can be diagnosed with a higher signal of hyperpolarized 1-$^{13}$C-glutamate from hyperpolarized 1-$^{13}$C-2-oxoglutarate. This result is in complete agreement with the results obtained in example 9 using conventional biochemical methods.

**Example 11 : Comparison of amount of signal produced on Hyperpolarized 1-$^{13}$C-lactate from Hyperpolarized 1-$^{13}$C-pyruvate and Hyperpolarized 1-$^{13}$C-glutamate from Hyperpolarized 1-$^{13}$C-2-oxoglutarate.**

30 μmol of a 1-$^{13}$C-2-oxoglutaric acid sample made according to example 2, and 30 μmol of a 1-$^{13}$C pyruvic acid sample, comprising 17 mM Ox063 radical and 2 mM Gadoteridol, were placed in the sample cup without mixing. The two samples were then hyperpolarized and dissolved in 5ml phosphate buffer (40 mM, pH 7.3) with addition of NaOH (7.5 μl, 12 M). The pH after dissolution was 7.3.

10 million liver cancer cells (Morris7777), were harvested and redissolved in 500 μl phosphate buffer (PBS). To this suspension was added 100 μl of a 250 mM aspartate solution and the mixture was placed in a 10 mm NMR tube and placed with a connecting tubing in a 14.1 T magnet at 37° C.

2 ml of the dissolved mixture of hyperpolarized 1-$^{13}$C-2-oxoglutarate and hyperpolarized 1-$^{13}$C-pyruvate was injected through the connecting line (dead volume 1 ml) resulting in a total substrate concentration of 3.5 mM. A series of 30 degree pulses every 2 s (56 scans in total) was acquired. The acquisition was started just before injection of the hyperpolarized substrates.

The result is shown in Figure 13 where it can be appreciated that the produced hyperpolarized signal of 1-$^{13}$C-glutamate from hyperpolarized 1-$^{13}$C-2-oxoglutarate is more than 2 times higher than the produced hyperpolarized signal of 1-$^{13}$C-lactate from hyperpolarized 1-$^{13}$C-pyruvate in liver cancer cells.

## Claims

1. A diagnostic Magnetic Resonance (MR) method performed on a subject who is affected or suspected to be affected by a tumor, said subject having been positioned in an MRI system, administered with hyperpolarized 1-$^{13}$C-2-oxoglutarate, and submitted to a radiation having a frequency selected to excite nuclear spin transitions in $^{13}$C nuclei; said method comprising:

   a. recording an MR signal from $^{13}$C excited nuclei of hyperpolarized 1-$^{13}$C-glutamate obtained from metabolic conversion of said hyperpolarized 1-$^{13}$C-2-oxoglutarate;
   b. comparing a first MR signal deriving from a region of interest comprising said tumor or said suspected tumor with a second MR signal deriving from said subject.

2. The method according to claim 1 wherein said hyperpolarized 1-$^{13}$C-2-oxoglutarate has been metabolically converted into hyperpolarized 1-$^{13}$C-glutamate through a reaction catalyzed by aspartate transaminase.

3. The method according to anyone of claims 1 or 2, further comprising the steps of:

   d. calculating a difference between said first signal and second signal;
   e. comparing said difference of step d) with a reference value, to produce a deviation value,
   f. comparing the deviation value with a predetermined value.

4. Hyperpolarized 1-$^{13}$C-2-oxoglutarate for use in the method according to claim 3, wherein said second signal is

determined on a non-tumor tissue, further comprising the steps of:
g. providing an indication of possible tumor affection in case the deviation value is in absolute value higher than said predetermined value.

**5.** Hyperpolarized 1-$^{13}$C-2-oxoglutarate for use in the method according to claim 3, wherein said second signal is determined in the region of interest at an earlier moment in time with respect to the first signal, and optionally stored in the system, further comprising the steps of:
g'. providing an indication of tumor variation in case the deviation is in absolute value higher than said predetermined value.

**6.** Hyperpolarized 1-$^{13}$C-2-oxoglutarate for use in the method according to claim 3, wherein said subject has undergone an anti-tumor treatment and wherein said second signal is determined in the region of interest, or on said second sample comprising said tumor-bearing or suspected tumor-bearing tissue, at an earlier moment in time with respect to said first signal further comprising the steps of:
g". providing an indication of efficacy of said treatment if this deviation is in absolute value higher than a predetermined value.

**7.** The method according to claim 3 wherein said second signal is determined shortly before, shortly after or at the beginning of the treatment.

**8.** The method according to anyone of claims 1-3, 7 or the hyperpolarized 1-$^{13}$C-2-oxoglutarate for use according to anyone of claims 4-6, wherein said subject has been co-administered unlabeled, non-polarized aspartate or glutamate when treated with hyperpolarized 1-$^{13}$C-2-oxoglutarate.

## Patentansprüche

**1.** Diagnostisches Magnetresonanz (MR)-Verfahren, das bei einem Subjekt durchgeführt wird, das von einem Tumor betroffen ist oder bei dem der Verdacht auf einen Tumor besteht, wobei das Subjekt in einem MRI-System positioniert wurde, dem Subjekt hyperpolarisiertes 1-$^{13}$C-2-Oxoglutarat verabreicht wurde, und das Subjekt einer Strahlung mit einer Frequenz ausgesetzt wurde, die ausgewählt ist, um Kernspin-Übergänge in $^{13}$C-Kernen anzuregen; wobei das Verfahren umfasst:

a. Aufzeichnen eines MR-Signals von $^{13}$C-angeregten Zellkernen von hyperpolarisiertem 1-$^{13}$C-Glutamat, das aus der metabolischen Umwandlung des hyperpolarisierten 1-$^{13}$C-2-Oxoglutarats erhalten wurde;
b. Vergleichen eines ersten MR-Signals, das von einer interessierenden Region abgeleitet wird, die den Tumor oder den vermuteten Tumor umfasst, mit einem zweiten MR-Signal, das aus dem Subjekt abgeleitet wird.

**2.** Verfahren nach Anspruch 1, wobei das hyperpolarisierte 1-$^{13}$C-2-Oxoglutarat metabolisch in hyperpolarisiertes 1-$^{13}$C-Glutamat umgewandelt wurde durch eine Reaktion, die durch Aspartat-Transaminase katalysiert wurde.

**3.** Verfahren nach einem der Ansprüche 1 oder 2, ferner umfassend die Schritte:

d. Berechnen einer Differenz zwischen dem ersten Signal und dem zweiten Signal;
e. Vergleichen der Differenz von Schritt d) mit einem Referenzwert, um einen Abweichungswert zu erzeugen,
f. Vergleichen des Abweichungswertes mit einem vorbestimmten Wert.

**4.** Hyperpolarisiertes 1-$^{13}$C-2-Oxoglutarat zur Verwendung in dem Verfahren nach Anspruch 3, wobei das zweite Signal für ein Nichttumorgewebe bestimmt wird, ferner umfassend den Schritt:
g. Bereitstellen einer Anzeige einer möglichen Tumoraffektion, falls der Abweichungswert ein absoluter Wert ist, der höher ist als der vorbestimmte Wert.

**5.** Hyperpolarisiertes 1-$^{13}$C-2-Oxoglutarat zur Verwendung in dem Verfahren nach Anspruch 3, wobei das zweite Signal in der interessierenden Region zu einem früheren Zeitpunkt mit Bezug auf das erste Signal bestimmt und optional in dem System gespeichert wird, ferner umfassend den Schritt:
g'. Bereitstellen einer Anzeige einer Tumorvariation, falls die Abweichung ein absoluter Wert ist, der höher ist als der vorbestimmte Wert.

**6.** Hyperpolarisiertes 1-$^{13}$C-2-Oxoglutarat zur Verwendung in dem Verfahren nach Anspruch 3, wobei das Subjekt einer Antitumorbehandlung unterzogen wurde und wobei das zweite Signal in der interessierenden Region oder für die zweite Probe, die tumortragendes oder vermutetes tumortragendes Gewebe umfasst, zu einem früheren Zeitpunkt mit Bezug auf das erste Signal bestimmt wird, ferner umfassend den Schritt:
g". Bereitstellen einer Anzeige der Wirksamkeit der Behandlung, wenn diese Abweichung ein absoluter Wert ist, der höher ist als ein vorbestimmter Wert.

**7.** Verfahren nach Anspruch 3, wobei das zweite Signal kurz vor, kurz nach oder zu Beginn der Behandlung bestimmt wird.

**8.** Verfahren nach einem der Ansprüche 1-3, 7 oder hyperpolarisiertes 1-$^{13}$C-2-Oxoglutarat zur Verwendung nach einem der Ansprüche 4-6, wobei dem Subjekt gleichzeitig unmarkiertes, nicht polarisiertes Aspartat oder Glutamat verabreicht wurde, wenn es mit hyperpolarisiertem 1-$^{13}$C-2-Oxoglutarat behandelt wird.

## Revendications

**1.** Procédé de diagnostic par résonance magnétique (RM) réalisé sur un sujet qui est affecté ou suspecté d'être affecté par une tumeur, ledit sujet ayant été positionné dans un système d'IRM, administré avec du 1-$^{13}$C-2-oxoglutarate hyperpolarisé, et soumis à un rayonnement ayant une fréquence choisie pour exciter des transitions de spin nucléaire dans des noyaux $^{13}$C ; ledit procédé comprenant :

a. l'enregistrement d'un signal RM en provenance de noyaux excités en $^{13}$C de 1-$^{13}$C-glutamate hyperpolarisé obtenu par conversion métabolique dudit 1-$^{13}$C-2-oxoglutarate hyperpolarisé ;
b. la comparaison d'un premier signal RM dérivant d'une région d'intérêt comprenant ladite tumeur ou ladite tumeur suspectée avec un second signal RM dérivant dudit sujet.

**2.** Procédé selon la revendication 1, ledit 1-$^{13}$C-2-oxoglutarate hyperpolarisé ayant été métaboliquement converti en 1-$^{13}$C-glutamate hyperpolarisé par une réaction catalysée par de l'aspartate transaminase.

**3.** Procédé selon l'une quelconque des revendications 1 ou 2, comprenant en outre les étapes de :

d. calcul d'une différence entre ledit premier signal et ledit second signal ;
e. comparaison de ladite différence de l'étape d) avec une valeur de référence, pour obtenir une valeur d'écart,
f. comparaison de la valeur d'écart avec une valeur prédéterminée.

**4.** 1-$^{13}$C-2-oxoglutarate hyperpolarisé destiné à être utilisé dans le procédé selon la revendication 3, ledit second signal étant déterminé sur un tissu non tumoral, comprenant en outre les étapes de :
g. fourniture d'une indication de l'affection possible de la tumeur dans le cas où la valeur d'écart en valeur absolue est supérieure à ladite valeur prédéterminée.

**5.** 1-$^{13}$C-2-oxoglutarate hyperpolarisé destiné à être utilisé dans le procédé selon la revendication 3, ledit second signal étant déterminé dans la région d'intérêt à un moment antérieur dans le temps par rapport au premier signal, et éventuellement stocké dans le système, comprenant en outre les étapes de :
g'. fourniture d'une indication de la variation de la tumeur dans le cas où l'écart en valeur absolue est supérieur à ladite valeur prédéterminée.

**6.** 1-$^{13}$C-2-oxoglutarate hyperpolarisé destiné à être utilisé dans le procédé selon la revendication 3, ledit sujet ayant subi un traitement antitumoral et ledit second signal étant déterminé dans la région d'intérêt, ou sur ledit second échantillon comprenant ledit tissu porteur d'une tumeur ou suspecté de porter une tumeur, à un moment antérieur dans le temps par rapport audit premier signal comprenant en outre les étapes de :
g". fourniture d'une indication de l'efficacité dudit traitement si cet écart en valeur absolue est supérieur à une valeur prédéterminée.

**7.** Procédé selon la revendication 3, ledit second signal étant déterminé peu avant, peu après ou au début du traitement.

**8.** Procédé selon l'une quelconque des revendications 1-3, 7 ou 1-$^{13}$C-2-oxoglutarate hyperpolarisé destiné à être utilisé selon l'une quelconque des revendications 4-6, de l'aspartate non polarisé ou du glutamate sans marquage

ayant été co-administré audit sujet, lorsqu'il est traité avec du 1-$^{13}$C-2-oxoglutarate hyperpolarisé.

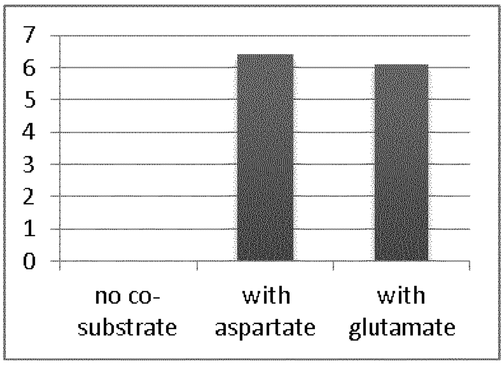


Figure 1

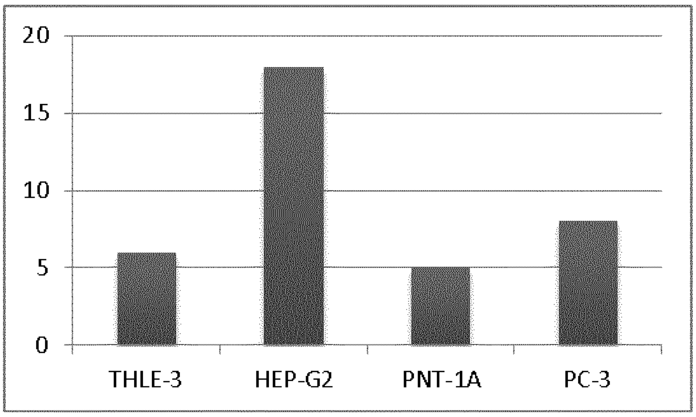


Figure 2

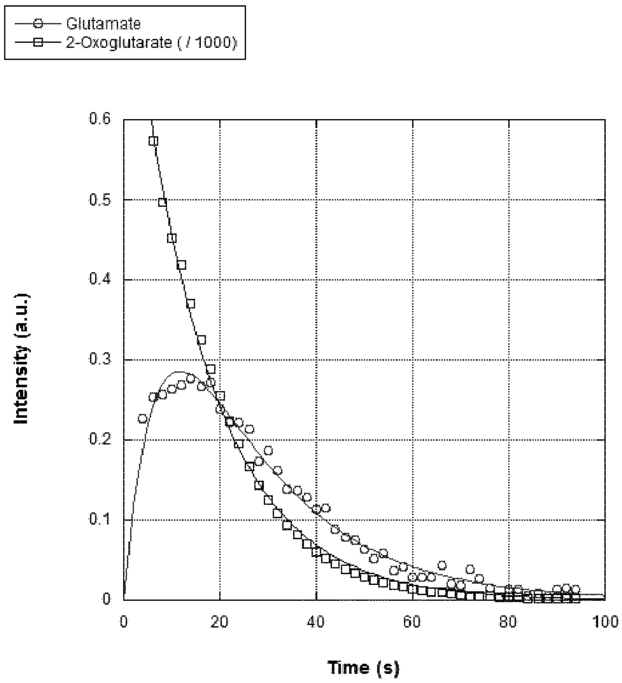


Figure 3

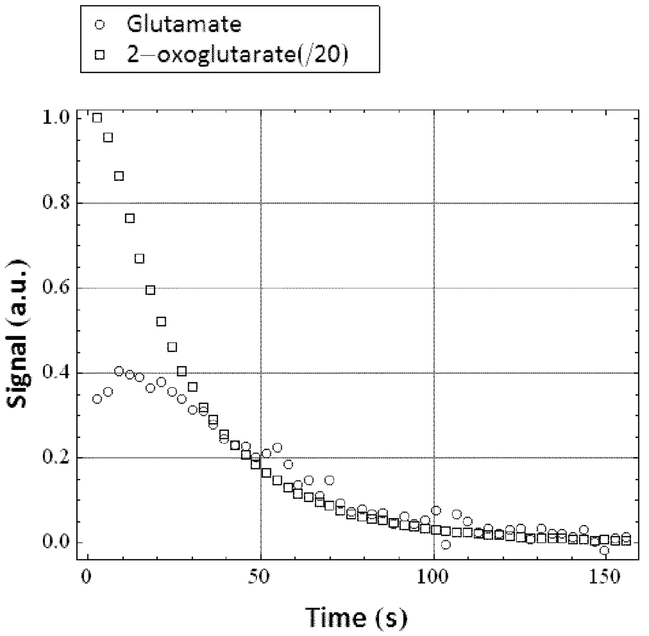


Figure 4

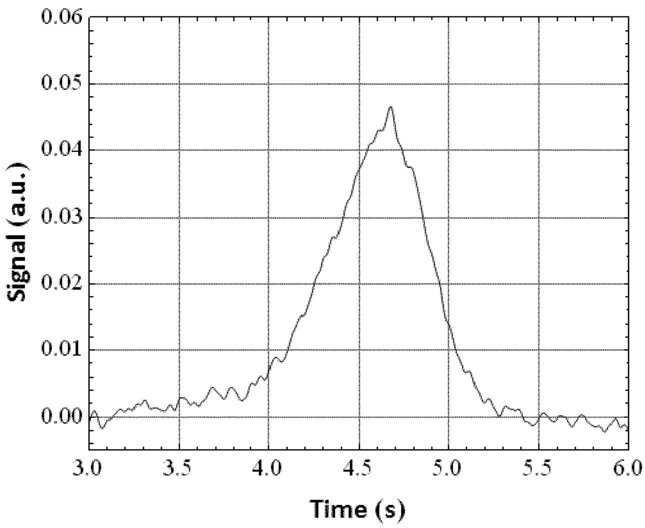


Figure 5

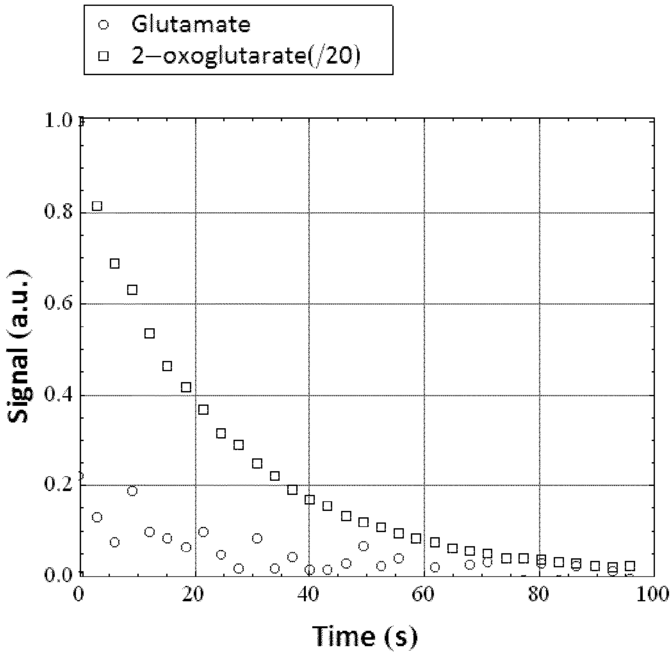


Figure 6

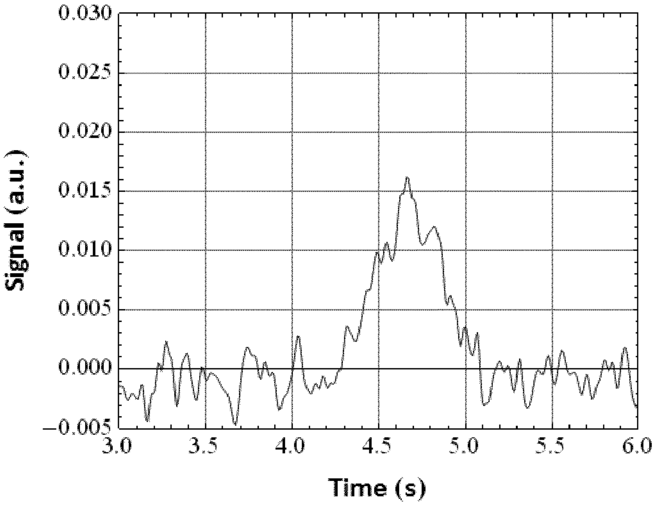


Figure 7

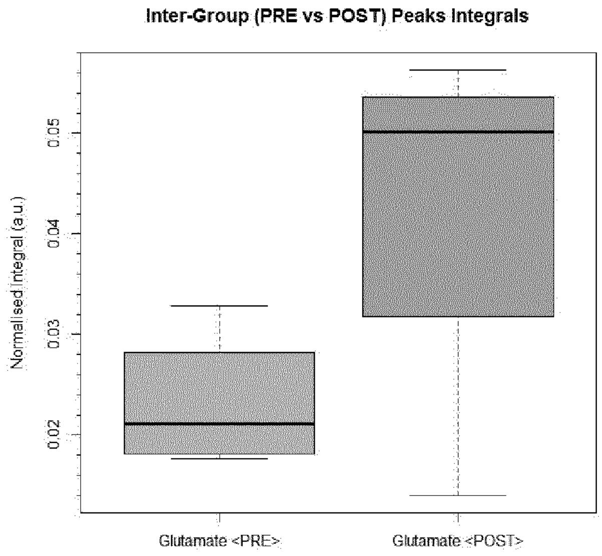


Figure 8

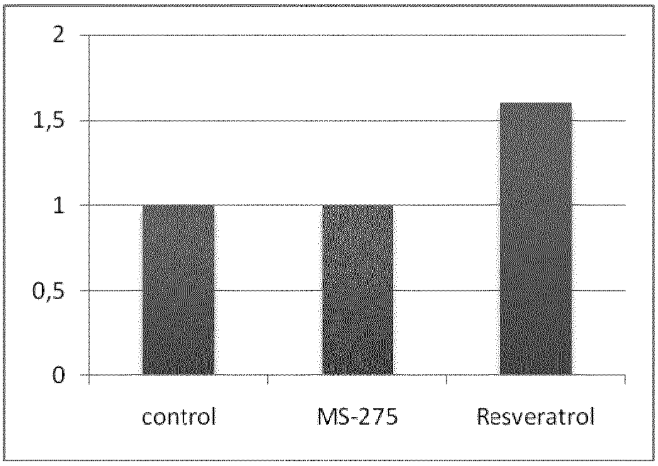


Figure 9

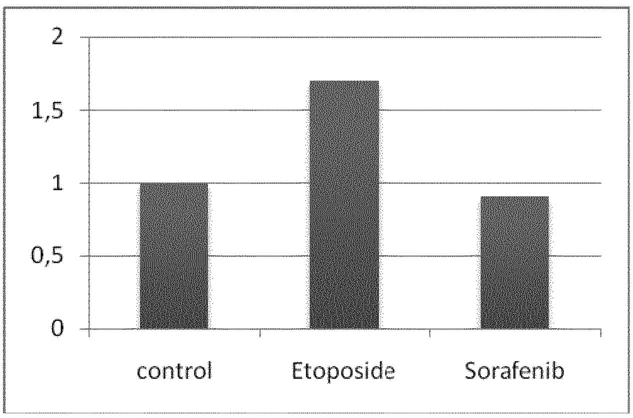


Figure 10

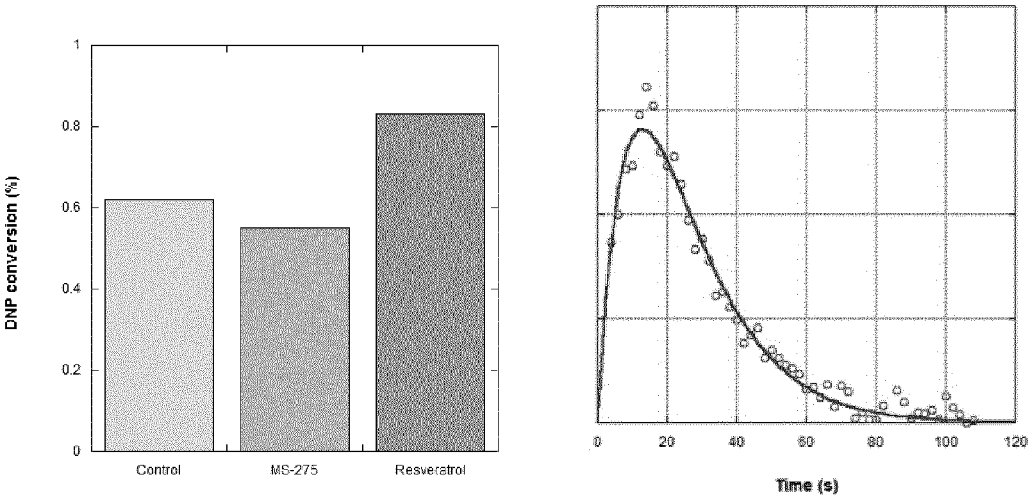


Figure 11

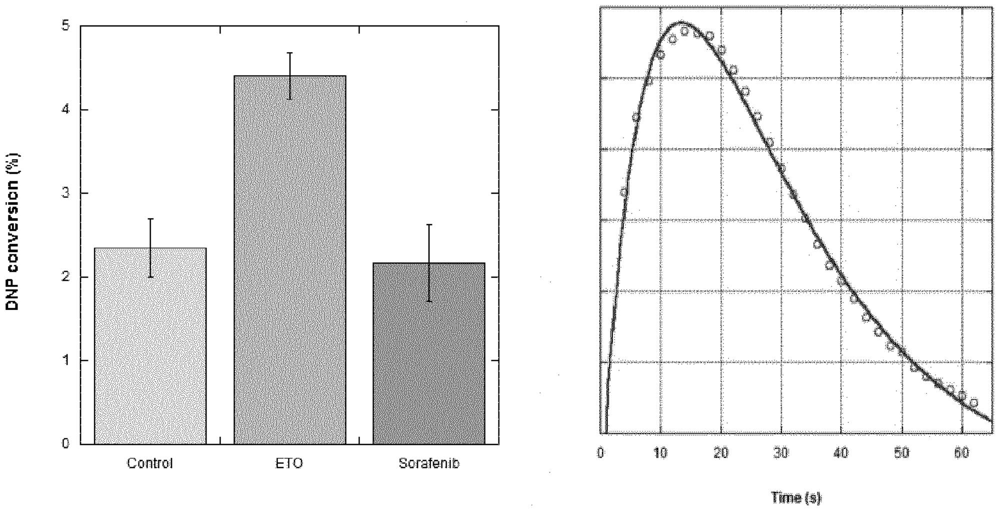


Figure 12

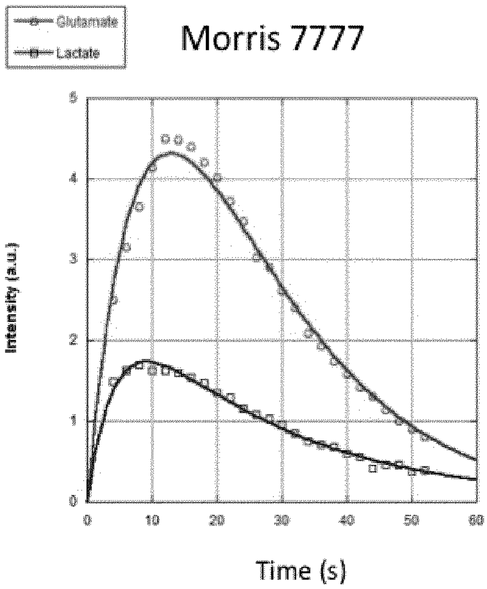
Glutamate
Lactate
Morris 7777
Intensity (a.u.)
Time (s)
0
1
2
3
4
5
0
10
20
30
40
50
60

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9935508 A
- WO 2009077575 A
- WO 2011124672 A
- WO 200977575 A


### Non-patent literature cited in the description


- **DAY SE ; KETTUNEN MI ; GALLAGHER FA ; HU DE ; LERCHE M ; WOLBER J ; GOLMAN K ; ARDENKJAER-LARSEN JH ; BRINDLE KM.** Detecting tumor response to treatment using hyperpolarized C magnetic resonance imaging and spectroscopy. *Nat Med,* 2007, vol. 13, 1382-1387
- **GALLAGHER FA ; KETTUNEN MI ; HU DE ; JENSEN PR ; ZANDT RI ; KARLSSON M ; GISSELSSON A ; NELSON SK ; WITNEY TH ; BOHNDIEK SE.** Production of hyperpolarized [1,4-13C2]malate from [1,4-13C2]fumarate is a marker of cell necrosis and treatment response in tumors. *Proc Natl Acad Sci USA,* 2009, vol. 106, 19801-19806
- **MERRITT ME ; HARRISON C ; STOREY C ; JEFFREY FM ; SHERRY AD ; MALLOY CR.** Hyperpolarized C allows a direct measure of flux through a single enzyme-catalyzed step by NMR. *Proc Natl Acad Sci USA,* 2007, vol. 104, 19773-19777
- **SCHROEDER MA ; SWIETACH P ; ATHERTON HJ ; GALLAGHER FA ; LEE P ; RADDA GK ; CLARKE K ; TYLER DJ.** Measuring intracellular pH in the heart using hyperpolarized carbon dioxide and bicarbonate: a C and P MRS study. *Cardiovasc Res,* 2010, vol. 86, 82-91
- **HU S ; CHEN AP ; ZIERHUT ML ; BOK R ; YEN YF ; SCHROEDER MA ; HURD RE ; NELSON SJ ; KURHANEWICZ J ; VIGNERON DB.** In vivo carbon-13 dynamic MRS and MRSI of normal and fasted rat liver with hyperpolarized C-pyruvate. *Mol Imaging Biol,* 2009, vol. 11, 399-407
- **GALLAGHER FA ; KETTUNEN MI ; DAY SE ; HU DE ; ARDENKJAER-LARSEN JH ; ZANDT R ; JENSEN PR ; KARLSSON M ; GOLMAN K ; LERCHE MH.** Magnetic resonance imaging of pH in vivo using hyperpolarized C-labelled bicarbonate. *Nature,* 2008, vol. 453, 940-943
- **GALLAGHER FA ; KETTUNEN MI ; DAY SE ; LERCHE M ; BRINDLE KM.** C MR spectroscopy measurements of glutaminase activity in human hepatocellular carcinoma cells using hyperpolarized C-labeled glutamine. *Magn Reson Med,* 2008, vol. 60, 253-257
- **JENSEN PR ; PEITERSEN T ; KARLSSON M ; IN 'T ZANDT R ; GISSELSSON A ; HANSSON G ; MEIER S ; LERCHE MH.** Tissue-specific short chain fatty acid metabolism and slow metabolic recovery after ischemia from hyperpolarized NMR in vivo. *J Biol Chem,* 2009, vol. 284, 36077-36082
- **KARLSSON M ; JENSEN PR ; ZANDT R ; GISSELSSON A ; HANSSON G ; DUUS JO ; MEIER S ; LERCHE MH.** Imaging of branched chain amino acid metabolism in tumors with hyperpolarized C ketoisocaproate. *Int J Cancer,* 2010, vol. 127, 729-736.10
- **ZHAO S ; LIN Y ; XU W ; JIANG W ; ZHA Z ; WANG P ; YU W ; LI Z ; GONG L ; PENG Y.** Glioma-derived mutations in IDH1 dominantly inhibit IDH1 catalytic activity and induce HIF-1alpha. *Science,* 2009, vol. 324, 261-265
- **FERDIA A. GALLAGHER ; MIKKO I. KETTUNEN ; SAM E. DAY ; DE-EN HU ; MAGNUS KARLSSON ; ANNA GISSELSSON ; MATHILDE H. LERCHE ; KEVIN M. BRINDLE.** Detection of Tumor Glutamate Metabolism In Vivo Using C Magnetic Resonance Spectroscopy and Hyperpolarized [1-13C]glutamate. *Magnetic Resonance in Medicine,* 2011, vol. 66, 18-23
- **MYRIAM MARIANNE CHAUMEIL ; SARAH WOODS ; ROBERT M DANFORTH ; HIKARI YOSHIHARA ; ALESSIA LODI ; AARON ROBINSON ; JOANNA J. PHILIPS ; SABRINA M RONEN.** Non-invasive assessment of IDH status in glioblastomas using dynamic C MRS of hyperpolarized $\alpha$-ketoglutarate. *Proc. Intl. Soc. Mag. Reson. Med.,* 2012, vol. 20